# EUROPEAN PATENT SPECIFICATION

(11) **EP 0 497 800 B1**
(45) Date of publication and mention of the grant of the patent: **28.12.1994**
(21) Application number: 90915284.5
(22) Date of filing: 26.10.1990
(51) Int. Cl.: A61K 7/06

(54) **COMPOSITION FOR HAIR GROWTH STIMULATION**
MITTEL ZUR ANREGUNG DES HAARWUCHSES
COMPOSITION STIMULANT LA CROISSANCE DES CHEVEUX

(30) Priority: 27.10.1989 US 427751
(43) Date of publication of application: 12.08.1992
(73) Proprietor: WANG, Yu, Kuei, Richard, Quebec, QC G2K 2G9 (CA); NGUYEN-DORAIS, Van, Dung, Quebec G1W 3N3 (CA)
(72) Inventor: WANG, Yu, Kuei, Richard, Quebec, QC G2K 2G9 (CA); NGUYEN-DORAIS, Van, Dung, Quebec G1W 3N3 (CA)
(74) Representative: Hubert, Philippe
(86) International application number: CA9000370
(87) International publication number: WO9106278

(56) References cited:
- WO-A-89/09047
- FR-A- 2 165 752
- FR-A- 2 299 019
- FR-A- 2 338 035
- STN File Supplier, Karlsruhe, DE, Chemical Abstracts, vol. 104, no. 2 (Colombus, Ohio, US), abstract 10372c & JP-A-60139612 (Lion Corp.)

## Description

The present invention relates to a topical composition for stimulating hair growth and/or preventing or decreasing hair loss.

### BACKGROUND OF THE INVENTION

Baldness also referred to as alopecia, is the result of partial or complete loss of hair. Baldness can be congenital or acquired, localized or generalized, temporary or definitive. Generally speaking, men are more affected by alopecia than women though it has been noticed that more women are subject to loss of hair probably due to the fact that they are subjected to the same stress.

Certain cases of alopecia are the result of different infections diseases, malnutrition, high fevers such as typhoid or pneumonia, surgical chock, trauma after delivery. On the other hand, the most frequent cause of alopecia is seborrhea accompanied with dry or oily dandruff, the origin of which is unknown even though it is believed that the secretion gland under the scalp plays an important role. In certain cases of alopecia, the cause is unknown.

There are several types of alopecia.

In alopecia "areata" sudden hair loss is usually circumscribed to certain areas and occurs in individuals who have no obvious skin disorder or systemic disease. Any hairy area may be involved, the scalp and beard most frequently and it is rare that all the body hair is lost (alopecia universalia).
Another form of alopecia is "scarring" alopecia where the hair loss is due to atrophy or scarring and thus where no regrowth can be expected. Scarring alopecia can be caused by any of the following: development failure, hereditary disorders, physical causes such as burning, mechanical traumatism and radioactive treatment, viral, bacterial, mushroom, pyrogenic and similar infections, neoplasm, and dermatosis of unknown causes.

Since alopecia in its various forms occurs in a certain percentage of the male population and because its existence is highly undesirable from an esthetic and psychological point of view, it would thus appear that it would be highly desirable if a preparation suitable for favoring the growth of hair to relieve alopecia could be found.

One product that has proven effective in stimulating hair growth is minoxidil. However, this product has the drawback that it must be administered continually as ceasing the administration results in subsequent hair loss.

It is thus highly desirable to provide a composition that can stimulate hair growth and whereby the new hair grown is not lost after the application of the composition is discontinued.

Document WO-A-89/09047 generally discloses a hair lotion containing 13 to 17% in volume of an alcoholic extract of cardamom.

On the other hand, FR-A-2 165 572 and FR-A-2 338 035 describe compositions for stimulating hair growth containing natural extracts of orange or of a lemon essential oil.

Although the compositions described in these prior art documents comprise, among a large number of components, limonene, there is no indication, nor even a remote suggestion that it is the limonene contained in these compositions which is responsible for the activity of the latter.

### SUMMARY OF THE INVENTION

It has been discovered that limonene alone was effective in stimulating hair growth and preventing hair loss.

This constitutes the basis of the invention.

Thus, the invention is primarily directed to the use of limonene as the active ingredient in a composition, for stimulating hair growth and preventing or decreasing hair loss.

In accordance with the present invention, there is now provided a composition for topical application for the stimulation of normal hair growth characterized in that the composition contains a therapeutically effective amount of limonene, as active ingredient.

In another aspect of the present invention, the composition for topical application for stimulation of hair growth contains a mixture of limonene, in association with an aqueous extract of human and/or animal placenta, the latter, because of its content is particularly adapted to increase the unexpected hair growing effects of the limonene by providing essential nutrient elements to the area where the composition is applied, and eventually to the newly grown hair.

### DETAILED DESCRIPTION

Limonene is a well known product described in the literature and is readily available on the market from Aldrich Chemical Co., Milwaukee, Wis. or Fluka Chemicals Corp., Ronkonkoma, N.Y.

Limonene is a naturally occurring product found in plants. In accordance with the present invention, limonene has been shown, unexpectingly and for the first time, to stimulate hair growth as well as diminish or prevent the loss of hair.

Limonene, since it is a liquid, can be used as such without further dilution. Nevertheless, from an economical point of view. it is normally used in a diluted form which can vary from 0.5 to 99% by volume, with a dermatologically acceptable solvent, with a range of 0.5 to 30% by volume being preferred. An example of a suitable solvent, there may be mentioned ethanol or 2-propanol and the like, or mixtures thereof. Water can also be used, alone or in mixtures with solvent.

It has also been found that hair growth can be further enhanced by mixing the limonene with a liquid hydrosoluble human or animal placental extract which contain nutrient elements and thus contribute to increase the amount and speed of hair growth. In this case, no solvent is used and the mixture must be shaken thoroughly before use.

When a combination of limonene and placental extract is used, the proportion of each may vary from 20 to 80% by volume of each component, with a range of from 40 to 60% by volume being preferred. For example, when 20% of limonene is used, it is mixed with 80% of placental extract, and vice-versa.

Alternatively, lower amounts of limonene and placental extract can be used in which case a diluent will be used as a carrier for the two ingredients. In such a case, between 20 and 35% by volume of limonene and 20 to 35% of placental extract is used, the remainder being a water-soluble dermatologically acceptable solvent or water which are used as diluents. Preferably the limonene and placental extract are used in equal amounts.

As an example of a suitable placental extract used for the purpose of the present invention, there may be mentioned the preparation manufactured by Chemische Laboratorium Dr. Kurt Richter, West Berlin.

Generally, 0.5 ml of the composition of the present invention is applied on an area in need of normal hair growth according to the size of the bald or partly bald area, or 2-3 drops on the eyebrows. In the case of a totally bald head, 1-2 ml are necessary. The application is done once or twice a day, after a shower, which takes off the greasy layer on the skin, thus favoring the absorption of the composition, and enhancing its effectiveness. Preferably, the area where the composition is applied is rubbed vigorously to further enhance the absorption of the composition.

To evaluate the progress regarding the enhancement of hair growth, a method of counting the hair has been used. A stereotaxic apparatus similar to one used in neurosurgery is employed to determine a specific bald area of the scalp. The hair in this defined area is counted before and after treatment. The quotient of the difference between the number of hair in the counted area after the treatment and the number of hair before the treatment, over the number of hair before the treatment, gives the percentage of enhancement of hair on the evaluated area.

The reason for the use of this technique is that this apparatus allows one to relocalize, after the treatment, exactly the same area in which the hair was counted before the beginning of the treatment by a system of coordinates specific to the size, the shape and the chosen area of the head. The total surface of the selected area for counting is generally 1.8 cm².

The following are examples of practice this invention for the purpose of illustration and not limitation.

### Examples

The initial, qualitative tests were performed on the inventors themselves using limonene alone. Their preliminary findings demonstrated that limonene was effective in stimulating hair growth and preventing hair loss. Subsequent to these positive findings, ten individuals were treated in two studies. The results follow.
The following solutions are prepared:
(concentration are given by volume)

| SOLUTION COMPOUNDS | 1 | 2 | 3 | 4 | 5 | 6 |
|---|---|---|---|---|---|---|
| limonene | 20 | 20 | 20 | 20 | 50 | 25 |
| hydrosoluble placenta | 5 | 10 | 15 | 15 | 50 | 25 |
| ethanol | 37.5 | 35 | 32.5 | 21.7 | -- | 25 |
| 2-propanol | 37.5 | 35 | 32.5 | 21.7 | -- | -- |
| distilled water | -- | -- | -- | 21.7 | -- | 25 |

The results are given for 3 different groups of individuals, said groups having each a defined treatment period.

### GROUP #1: 8-9 months treatment.

All individuals in this group (6) are men aging from 31 to 54.
In all cases, except cases 1 and 4, the cause of the baldness was unknown. In case 1, the cause was hereditary and in case 2, it was due to stress and/or fatigue. Each was treated during 4 months with solution #1, then 2.5 months with solution #2. The duration of the treatment with solution #3 was variable, depending on the case.

| Case # | # Years bald | Treatment duration with solution #3 | Percentage of enhancement of hair growth after after the treatment |
|---|---|---|---|
| 1 | 13 | 2.25 months | 43.85 |
| 2 | 18 | 2.50 months | 170.96 |
| 3 | 33 | 2.25 months | 83.33 |
| 4 | 18 | 2.00 months | 2,100.00 |
| 5 | 5 | 2.50 months | 229.00 |
| 6 | 16 | 2.00 months | downy surface |

Similar results were obtained with solution #1, #2 and #4.

### GROUP #2: 3-5 months treatment.

This group involved 3 men and a woman, aging from 32 to 55. The cause of baldness was unknown in the three men, cases 1-3. The woman, case 4, had a congenital absence of eyebrows. Each man was treated for 4 months with solution #1. The woman had her eyebrows treated with a solution of 3% of limonene alone in ethanol for 3 months. 2 of the 3 men received further treatment with solution #2 for a period of 0.5 month.

| Case # | Years bald | Duration of treatment with solution (months) | | Sex | Percentage of enhancement of hair after the treatment |
|---|---|---|---|---|---|
| | | #1 | #2 | | |
| 1 | 18-23 | 4 | - | M | 212.50 |
| 2 | 25 | 4 | 0.5 | M | 562.50 |
| 3 | 10 | 4 | 0.5 | M | 8.33 |
| 4 | 40 | - | - | W | eyebrows visible |

Similar results were obtained when using solution #5. Also, solution #6 has been found to be ideal for sensitive and/or dry skin.

It is to be noted that after the preliminary trials on themselves, the inventors ceased the application of limonene. The hair grown during the treatment still remains present after two years.

Of the individuals tested in the study, case 2 agreed to stop the treatment and his newly grown hair is still present after one and a half years.

## Claims

1. Use of limonene as the active ingredient in a composition for stimulating hair growth and preventing or decreasing hair loss.

2. Use as claimed in claim 1, wherein said composition further comprises a dermatologically acceptable solvent selected from the group consisting of ethanol, 2-propanol, water, and mixtures thereof.

3. Use as claimed in claim 1, wherein said composition further comprises a nutrient element selected from the group consisting of animal hydrosoluble placenta and/or human hydrosoluble placenta.

4. Use as claimed in claim 1, wherein said composition further comprises a nutrient element selected from the group consisting of animal hydrosoluble placenta and/or human hydrosoluble placenta, and a dermatologically acceptable diluent selected from the group consisting of ethanol, 2-propanol, water, and mixtures thereof.

5. Use as claimed in claim 2, wherein the concentration of limonene ranges from 0.5 to 99% by volume.

6. Use as claimed in claim 3, wherein the concentration of limonene ranges from 20 to 80% by volume, and wherein the concentration of the nutrient element ranges from 80 to 20% by volume.

## Patentansprüche

1. Verwendung von Limonen als aktiver Bestandteil in einer Zusammensetzung zur Stimulierung des Haarwuchses und zur Verhinderung oder Verringerung von Haarausfall.

2. Verwendung nach Anspruch 1, wobei die Zusammensetzung ferner ein dermatologisch annehmbares Lösungsmittel, ausgewählt aus der Gruppe bestehend aus Ethanol, 2-Propanol, Wasser und Mischungen hievon, umfaßt.

3. Verwendung nach Anspruch 1, wobei die Zusammensetzung ferner ein Nährstoffelement, ausgewählt aus der Gruppe bestehend aus tierischer hydrolöslicher Placenta und/oder menschlicher hydrolöslicher Placenta, umfaßt.

4. Verwendung nach Anspruch 1, wobei die Zusammensetzung ferner ein Nährstoffelement, ausgewählt aus der Gruppe bestehend aus tierischer hydrolöslicher Placenta und/oder menschlicher hydrolöslicher Placenta, und ein dermatologisch annehmbares Verdünnungsmittel, ausgewählt aus der Gruppe bestehend aus Ethanol, 2-Propanol, Wasser und Mischungen hievon, umfaßt.

5. Verwendung nach Anspruch 2, wobei die Konzentration von Limonen im Bereich von 0,5 bis 99 Vol.% liegt.

6. Verwendung nach Anspruch 3, wobei die Konzentration von Limonen im Bereich von 20 bis 80 Vol.% liegt, und wobei die Konzentration des Nährstoffelements im Bereich von 80 bis 20 Vol.% liegt.

## Revendications

1. Utilisation de limonène comme principe actif dans une composition pour stimuler la pousse des cheveux et prévenir ou ralentir la chute des cheveux.

2. Utilisation selon la revendication 1, dans laquelle ladite composition comprend en outre un solvant dermatologiquement acceptable, sélectionné dans le groupe constitué par l'éthanol, le propanol-2, l'eau et leurs mélanges.

3. Utilisation selon la revendication 1, dans laquelle ladite composition comprend en outre un élément nutritif sélectionné dans le groupe constitué par un placenta animal hydrosoluble et/ou un placenta humain hydrosoluble.

4. Utilisation selon la revendication 1, dans laquelle ladite composition comprend en outre un élément nutritif sélectionné dans le groupe constitué par un placenta animal hydrosoluble et/ou un placenta humain hydrosoluble, et un diluant dermatologiquement acceptable, sélectionné dans le groupe constitué par l'éthanol, le propanol-2, l'eau et leurs mélanges.

5. Utilisation selon la revendication 2, dans laquelle la concentration de limonène est comprise entre 0,5 et 99 % en volume.

6. Utilisation selon la revendication 3, dans laquelle la concentration de limonène est comprise entre 20 et 80 % en volume et dans laquelle la concentration de l'élément nutritif est comprise entre 80 et 20 % en volume.
